Europäisches Patentamt

European Patent Office

Office européen des brevets

(19)

(11) Veröffentlichungsnummer: **0 028 014**
**A1**

# (12) EUROPÄISCHE PATENTANMELDUNG

(21) Anmeldenummer: 80106493.2

(22) Anmeldetag: 23.10.80

(51) Int. Cl.³: **A 61 B 6/02**

(30) Priorität: 29.10.79 DE 2943643

(43) Veröffentlichungstag der Anmeldung: 06.05.81
Patentblatt 81/18

(84) Benannte Vertragsstaaten: DE FR GB IT SE

(71) Anmelder: SIEMENS AKTIENGESELLSCHAFT Berlin
und München, Postfach 22 02 61,
D-8000 München 22 (DE)

(72) Erfinder: Pfeiler, Manfred, Dr. Dipl.-Ing.,
Ludwig-Thoma-Strasse 25, D-8520 Erlangen (DE)
Erfinder: Pauli, Karlheinz, Dr. Dipl.-Phys., Würzburger
Ring 35, D-8520 Erlangen (DE)

(54) Schichtgerät zur Herstellung von Transversalschichtbildern.

(57) Die Erfindung bezieht sich auf einen Computer-Tomographen mit einer Strahlenmessanordnung, die zwei Röntgenröhren (1, 2) unterschiedlicher Strahlenenergie, welche
das Aufnahmeobjekt (4) durchdringende Strahlenbündel (7,
8) erzeugen, sowie einen Strahlenempfänger (5) enthält,
der die Strahlungsintensität hinter dem Objekt (4) ermittelt.
Das Aufnahmeobjekt (4) wird aus verschiedenen Richtungen durchstrahlt. Ein Rechner (9) bestimmt für jeden Bildpunkt den Schwächungskoeffizienten für jede Strahlenenergie und daraus die mittlere Ordnungszahl und die
Dichte. Der Strahlenempfänger (5) ist als feststehender
Detektorring ausgebildet. Zur Abtastung werden nur die
Röntgenröhren (1, 2) um das Aufnahmeobjekt (4) gedreht.
Sie sind in einem solchen Abstand voneinander gehalten,
dass kein Detektorelement gleichzeitig von beiden Strahlenbündeln getroffen wird.

SIEMENS AKTIENGESELLSCHAFT     Unser Zeichen
Berlin und München             VPA 79 P 5104 EUR


<u>Schichtgerät zur Herstellung von Transversalschicht-</u>
<u>bildern</u>


Die Erfindung betrifft ein Schichtgerät zur Herstellung von Transversalschichtbildern eines Aufnahmeobjektes mit einer Strahlenmeßanordnung, die zwei Strahlungsquellen unterschiedlicher Strahlenenergie, welche das Aufnahmeobjekt durchdringende Strahlenbündel erzeugen, deren Querschnittsausdehnung senkrecht zur Schichtebene im wesentlichen gleich der Schichtstärke ist, sowie einen Strahlenempfänger enthält, der die Strahlungsintensität hinter dem Objekt ermittelt, sowie mit einer Drehvorrichtung zur Durchstrahlung des Aufnahmeobjektes aus verschiedenen Richtungen und mit einem Rechner für die Bestimmung der Schwächungskoeffizienten jedes Bildpunktes in einer in der untersuchten Schicht liegenden Bildpunktmatrix, bei dem der Rechner so ausgebildet ist, daß er für jeden Bildpunkt den Schwächungskoeffizienten für jede Strahlenenergie und daraus die mittlere Ordnungszahl und die Dichte bestimmt.

Tp 5 Kli / 10.10.1979

0028014

Es ist ein Computer-Tomograph dieser Art bekannt, bei dem zur Abtastung des Objektes für jede Strahlungsquelle je ein Strahlenempfänger vorhanden ist, der mit der zugehörigen Strahlungsquelle starr verbunden ist (DE-AS 23 39 758). Zur Abtastung des Objektes erfolgt wechselweise eine gemeinsame seitliche Verschiebung beider Strahlungsquellen und beider Strahlenempfänger und eine Drehung dieser Bauelemente. Nachteilig ist bei dem bekannten Schichtgerät, daß der technische Aufwand für die Abtastung des Aufnahmeobjektes sehr groß ist.

Der Erfindung liegt die Aufgabe zugrunde, ein Schichtgerät der eingangs genannten Art so auszubilden, daß der Aufwand gegenüber dem Stand der Technik verringert ist.

Diese Aufgabe ist erfindungsgemäß dadurch gelöst, daß der Strahlenempfänger als feststehender Ring aus einer Vielzahl von Detektorelementen besteht und zur Abtastung nur die Strahlungsquellen um das Aufnahmeobjekt gedreht werden und daß die Strahlungsquellen in einem solchen Abstand voneinander gehalten sind, daß kein Detektorelement gleichzeitig von beiden Strahlenbündeln getroffen wird. Bei dem erfindungsgemäßen Schichtgerät werden zur Abtastung des Aufnahmeobjektes lediglich die beiden Strahlungsquellen um das Aufnahmeobjekt gedreht. Der als Detektorring ausgebildete Strahlenempfänger ist ortsfest. Jede Strahlungsquelle kann dabei ein fächerförmiges Strahlenbündel aussenden, dessen Randstrahlen die Randstrahlen des Meßfeldes bilden, so daß eine seitliche Verschiebung zur Abtastung der gesamten abzubildenden Schicht nicht erforderlich ist.

0028014

- 3 -    VPA 79 P 5104 EUR

Die Erfindung ist nachfolgend anhand eines in der Zeichnung dargestellten Ausführungsbeispiels näher erläutert. Es zeigen:

Fig. 1 ein Schichtgerät nach der Erfindung und

Fig. 2 eine Kurve zur Erläuterung der Fig. 1.

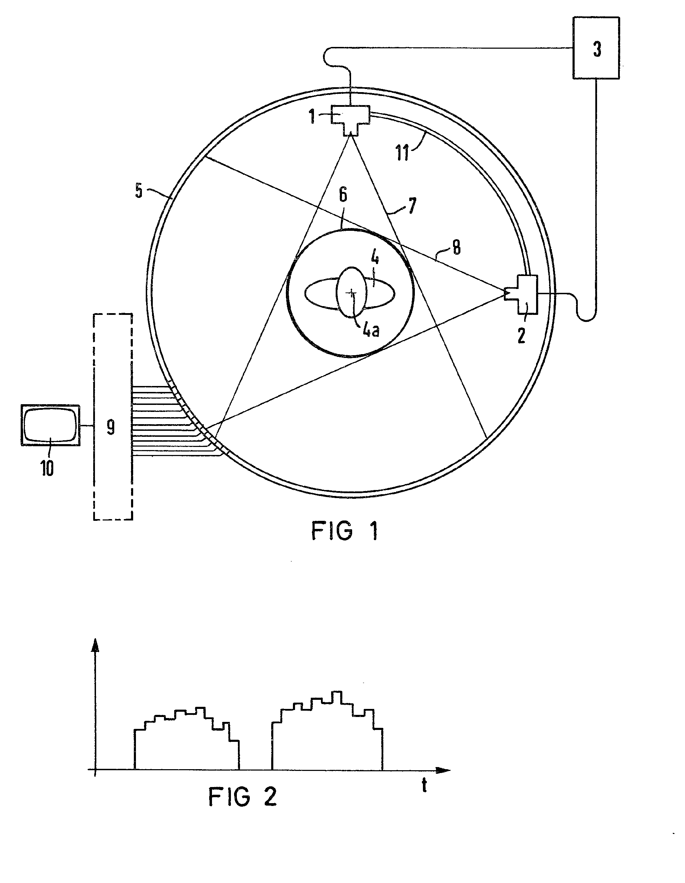

In der Fig. 1 sind zwei Röntgenröhren 1 und 2 dargestellt, die von einem Röntgengenerator 3 gespeist werden. Der Patient 4 wird von einem Strahlenempfänger 5 umschlossen, der aus einer Vielzahl von Detektorelementen für Röntgenstrahlung besteht. Der Strahlenempfänger 5 empfängt jeweils gleichzeitig zwei das Meßfeld 6 und damit eine Querschicht des Patienten 4 durchsetzende Strahlenfächer 7 und 8, deren Randstrahlen die Randstrahlen des Meßfeldes 6 bilden. Die Dicke der Strahlenfächer 7 und 8 ist senkrecht zur Schichtebene gleich der Schichtstärke. Jedes Detektorelement des Strahlenempfängers 5 ist an einem Rechner 9 angeschlossen, der aus den Ausgangssignalen der Detektorelemente des Strahlenempfängers 5, die bei der Drehung der Röntgenröhren 1 und 2 um den Patienten 4, und zwar um eine Achse 4a, entstehen, ein Bild der durchstrahlten Querschicht des Patienten 4 berechnet und seine Wiedergabe auf einem Sichtgerät 10 bewirkt. Die Bilderzeugung erfolgt dadurch, daß der Rechner die Schwächungskoeffizienten von Bildpunkten in einer in der untersuchten Schicht des Aufnahmeobjektes 5 liegenden Bildpunktmatrix bestimmt.

Die Röntgenröhren 1 und 2 werden mit unterschiedlichen Hochspannungen versorgt und liefern daher unterschiedliche Strahlenenergien. Der Rechner 9 berechnet für jede Strahlenenergie und jeden Bildpunkt den Schwächungskoeffizienten, so daß für jeden Bildpunkt zwei
Schwächungskoeffizienten vorliegen, aus denen die mittlere Ordnungszahl des Stoffes im Bildpunkt und die
Dichte berechnet werden kann. Die gesamte Abtastung
ist beendet, wenn beide Röntgenröhren 1, 2 sich um
360° um den Patienten 4 gedreht haben.

Der Strahlenempfänger 5, der als Detektorring ausgebildet ist, ist ortsfest. Die Röntgenröhren 1, 2 sind
durch eine Stange 11 in einem festen Abstand voneinander
gehalten, der so gewählt ist, daß kein Detektorelement
des Strahlenempfängers 5 gleichzeitig von den beiden
Strahlenfächern 7, 8 oder von Teilen davon getroffen
wird. Es ist daher möglich, mit nur einem Detektorsystem gleichzeitig zwei zur Rekonstruktion von zwei
Bildern benötigte Datensätze mit zwei verschiedenen
effektiven Energien aufzunehmen. Die beiden Datensätze werden zwar bei einem Abtastvorgang gewonnen,
jedoch werden von beiden Strahlenfächern 7, 8 immer
zwei verschiedene Bereiche des Strahlenempfängers 5
bestrahlt, so daß jedes Detektorelement nicht gleichzeitig, sondern nacheinander von beiden Strahlenfächern 7, 8 getroffen wird.

Die zeitliche Folge der von einem Detektorelement registrierten Meßwerte zeigt in einem Beispiel die Fig.2.
Man erhält zwei zeitlich getrennte Signale, die vor
der Bildrekonstruktion durch den Rechner 9 getrennt
bzw. sortiert werden können.

- 1 -

Patentansprüche

1. Schichtgerät zur Herstellung von Transversalschichtbildern eines Aufnahmeobjektes mit einer Strahlenmeß-anordnung, die zwei Strahlungsquellen unterschiedli-cher Strahlenenergie, welche das Aufnahmeobjekt durchdringende Strahlenbündel erzeugen, deren Querschnittsausdehnung senkrecht zur Schichtebene im wesentlichen gleich der Schichtstärke ist, sowie einen Strahlenempfänger enthält, der die Strahlungsintensität hinter dem Objekt ermittelt, sowie mit einer Drehvorrichtung zur Durchstrahlung des Aufnahmeobjektes aus verschiedenen Richtungen und mit einem Rechner für die Bestimmung der Schwächungskoeffizienten jedes Bildpunktes in einer in der untersuchten Schicht liegenden Bildpunktmatrix, bei dem der Rechner so ausgebildet ist, daß er für jeden Bildpunkt den Schwächungskoeffizienten für jede Strahlenenergie und daraus die mittlere Ordnungszahl und die Dichte bestimmt, d a d u r c h g e k e n n z e i c h n e t , daß der Strahlen-empfänger (5) als feststehender Ring aus einer Vielzahl von Detektorelementen besteht und zur Abtastung nur die Strahlungsquellen (1, 2) um das Aufnahmeobjekt (4) gedreht werden und daß die Strahlungsquellen (1, 2) in einem solchen Abstand voneinander gehalten sind, daß kein Detektorelement gleichzeitig von beiden Strahlenbündeln (7, 8) getroffen wird.

2. Schichtgerät nach Anspruch 1, d a d u r c h g e k e n n z e i c h n e t , daß jede Strahlungs-quelle (1, 2) ein fächerförmiges Strahlenbündel (7,8) aussendet, dessen Randstrahlen die Randstrahlen des Meß-feldes (6) bilden.

FIG 1

FIG 2

## EINSCHLÄGIGE DOKUMENTE

| Kategorie | Kennzeichnung des Dokuments mit Angabe, soweit erforderlich, der maßgeblichen Teile | betrifft Anspruch |
|-----------|-----------------------------------------------------------------------------------|-------------------|
| A | FR - A - 2 239 975 (SIEMENS A.G.) <br> * Seite 3, Zeile 32 - Seite 6, Zeile 17; Abbildungen 1,2 * | 1 |
| D | & DE - B - 2 339 758 | |
| | US - A - 3 435 220 (A.F. HANKEN/ INDUSTRIAL NUCLEONICS CORP.) <br> * Spalte 1, Zeilen 14-23; Spalte 2, Zeilen 8-16; Spalte 8, Zeile 24 - Spalte 10, Zeile 19; Abbildung 11 * | 1 |
| | US - A - 3 500 446 (K. HASEGAWA et al./ TOA NENRYO KOGYO K.K.) <br> * Spalte 3, Zeile 63 - Spalte 4, Zeile 59; Abbildung 1 * | 1 |
| | US - A - 4 066 900 (C.A.G. LE MAY/ EMI LTD.) <br> * Zusammenfassung; Spalte 5, Zeile 51 - Spalte 6, Zeile 43; Abbildung 3 * | 1,2 |
| | GB - A - 2 007 457 (AMERICAN SCIENCE & ENGINEERING, INC.) <br> * Zusammenfassung; Seite 1, Zeilen 79-107; Abbildung 1 * | 1,2 |
| | FR - A - 2 368 930 (SIEMENS AG) <br> * Seite 3, Zeilen 7-33; Seite 5, Zeilen 30-35; Seite 6, Zeilen 27-29; Abbildung 1 * | 1,2 |

./.

X | Der vorliegende Recherchenbericht wurde für alle Patentansprüche erstellt.

| Recherchenort | Abschlußdatum der Recherche | Prüfer |
|---------------|----------------------------|--------|
| Den Haag | 29-01-1981 | RIEB |

**KLASSIFIKATION DER ANMELDUNG (Int. Cl.³)**

A 61 B 6/02

**RECHERCHIERTE SACHGEBIETE (Int. Cl.³)**

A 61 B 6/02

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

EPA form 1503.1  06.78

**Europäisches Patentamt**

**EUROPÄISCHER RECHERCHENBERICHT**

| Kategorie | Kennzeichnung des Dokuments mit Angabe, soweit erforderlich, der Maßgeblichen Teile | betrifft Anspruch | KLASSIFIKATION DER ANMELDUNG (Int. Cl.³) |
|---|---|---|---|
| | **EINSCHLÄGIGE DOKUMENTE** | | |
| P | <u>FR - A - 2 424 017</u> (GENERAL ELEC-TRIC CO.)<br><br>* Seite 4, Zeile 32 - Seite 5, Zeile 3; Seite 5, Zeilen 23-30; Seite 7, Zeilen 21-33; Abbildungen 2,4 * <br><br>-- | 1,2 | |
| P | <u>DE - A - 2 832 459</u> (KOCH & STERZEL GmbH CO.)<br><br>* Seite 5, Zeile 8 - Seite 7, Zeile 5 *<br><br>---- | 1 | RECHERCHIERTE SACHGEBIETE (Int. Cl.³) |